# EUROPÄISCHE PATENTSCHRIFT

(11) **EP 3 474 938 B1**
(45) Veröffentlichungstag und Bekanntmachung des Hinweises auf die Patenterteilung: **05.08.2020**
(21) Anmeldenummer: 17749367.3
(22) Anmeldetag: 21.06.2017
(51) Int. Cl.: A61M 25/01, A61B 1/00, A61B 18/24, A61J 1/03, A61M 25/09, G02B 23/24, A61B 18/00, A61B 34/30

(54) **GERÄT ZUM GESTEUERTEN BEFÖRDERN EINES KATHETERS, LICHTLEITERS ODER KABELS**
APPLIANCE FOR CONVEYING A CATHETER, LIGHT GUIDE OR CABLE IN A CONTROLLED MANNER
APPAREIL D'ACHEMINEMENT COMMANDÉ D'UN CATHÉTER, D'UN GUIDE D'ONDES OU D'UN CÂBLE

(30) Priorität: 28.06.2016 CH 8232020
(43) Veröffentlichungstag der Anmeldung: 01.05.2019
(73) Patentinhaber: Pieper, Karl, 5702 Niederlenz (CH); Schmuck, Thilo, 6288 Schongau (CH)
(72) Erfinder: Pieper, Karl, 5702 Niederlenz (CH); Schmuck, Thilo, 6288 Schongau (CH)
(74) Vertreter: Felber, Josef
(86) Internationale Anmeldenummer: PCT/EP2017/065305
(87) Internationale Veröffentlichungsnummer: WO 2018/001836

(56) Entgegenhaltungen:
- WO-A1-93/20876
- WO-A1-2012/037213
- NL-C2- 1 019 350

## Beschreibung

Diese Erfindung betrifft ein Gerät, um einen Katheter in Form eines Lichtleiters, also eines Glasfaserkabels, ganz gezielt aus einem Patienten, an welchem der Katheter eingesetzt wird, zurückzuziehen. Indessen kann das Gerät auch für jegliche Situationen, in denen ein auch anders gearteter Katheter, ein Lichtleiter bzw. ein Glasfaserkabel oder auch ein sonstiges Kabel mit einem ganzen bestimmten Tempo genau kontrolliert und überwacht befördert werden soll, eingesetzt werden.

Ein besonderer Fall, welchem hier die Aufgabenstellung zugrunde lag, wird im Folgenden kurz erläutert. Venen sind Blutgefässe, welche das verbrauchte, sauerstoffarme Blut aus dem Gewebe zum Herz zurück transportieren. Um diesen Transport auch gegen die Schwerkraft zu bewältigen, sind die Venen in den Extremitäten mit Venenklappen ausgestattet, welche eine Ventilfunktion besitzen und so ein Zurückfliessen des Blutes in den Venen verhindern. Diese Venen in den Beinen und Armen des Menschen verlaufen teils innerhalb eines Muskels, teils aber auch im umliegenden Gewebe. Während jene innerhalb eines Muskels sich nicht über Gebühr ausdehnen können, so ist das bei jenen der Fall, welche in weichem Gewebe verlaufen. Bei vielen Menschen bilden sich vor allen Dingen an den Beinen sogenannte Krampfadern. Dies sind krankhaft erweiterte Venen, die die Schliessfähigkeit der Venenklappen verloren haben. Es kommt so zu einem Rückwärtsfliessen des Blutes in diesen krankhaft veränderten Venen, die dadurch ihre Transportfunktion verlieren und das Blut in das Gewebe zurück stauen. Dies geht meistens mit einer Verschlechterung der Blutzirkulation einher und verursacht häufig Beschwerden. Man schätzt, dass 25% der Menschen in der westlichen Welt im Laufe ihres Lebens mindestens einmal an ihren Venen operiert werden müssen. Dies geschah bis vor kurzem in den meisten Fällen durch Entfernen der betroffenen Venenabschnitte. In den letzten 2 Jahrzehnten wurden jedoch neuere Verfahren entwickelt, bei denen die zumindest die Hauptvene, die sogenannte Stammvene, nicht mehr entfernt wird, sondern im Körper belassen wird und statt dessen verschlossen wird. Hierbei haben Katheter, welche die Venen mit thermischer Energie verschliessen, die grösste Verbreitung gefunden. Es existieren verschiedene Kathetermodelle auf dem Markt, welche unterschiedliche Energien zum Erzeugen der Hitze einsetzen, welche schlussendlich die Vene verschweisst. Grosse Verbreitung haben Katheter gefunden, welche Laserlicht als Energiequelle einsetzen. Diese Katheter werden in die Vene eingeführt und ultraschallkontrolliert exakt in dem zu behandelnden Venensegment platziert. Die Vene wird danach, ebenfalls unter Ultraschallkontrolle, mit einer gekühlten Flüssigkeit umspritzt, welche dafür sorgt, dass die zu behandelnde Venenwand dem Laserkatheter unmittelbar anliegt und andererseits das umliegende Gewebe vor der Laserenergie geschützt wird. Der Katheter wird nach Aktivierung des Lasers in gleichmässiger Geschwindigkeit Millimeter für Millimeter zurückgezogen. Die hierfür einzusetzende Energie wird vor dem Eingriff anhand des mit Ultraschall gemessenen Venendurchmessers berechnet. Damit die berechnete Energie korrekt auf die Venenwand übertragen wird, sollte der Rückzug des Katheters sehr sorgfältig und mit gleichförmiger Geschwindigkeit durchgeführt werden, andernfalls wäre die auf ein bestimmtes Venensegment eingesetzte Energie entweder zu hoch oder aber zu niedrig. Dieses kann im ersteren Fall zu einer Perforation der Vene oder im zweiten Fall zu einem fehlenden Verschluss der Vene führen.

Am vorderen Ende des Katheters ist dieser mit einem Prisma ausgerüstet, welches dafür sorgt, dass der Laser allseits um ca. 90° abgelenkt wird und somit rund um die Achse des Lichtleiters abstrahlt. Damit kann er seine Energie rundum auf das Gewebe in der Vene aufbringen. Wird dann gleichzeitig von aussen um den Umfangsbereich der Vene Wasser in das Gewebe eingespritzt, so erzeugt das einen Auflagedruck rund um die Vene. Sie wird allseits radial zusammengedrückt und mittels der Laserenergie lassen sich die Innenseiten der Vene verschweissen und somit verschliessen. Man spricht von einer Venenverödung. Während der Laser aktiv ist, muss der Katheter ganz langsam aus der Vene zurückgezogen werden, mit einer möglichst gleichförmigen Geschwindigkeit von ca. 1mm/s. Zum Zuschweissen einer Vene bzw. einer Krampfader von 600mm Länge benötigt man daher für das Zurückziehen 600 Sekunden bzw. 10 Minuten. Das Herausziehen ist für den operierenden Chirurgen eine heikle Arbeit, die bisher von Hand bewältigt werden muss. Wird der Katheter nicht mit ganz gleichförmiger Geschwindigkeit aus der Vene gezogen, so droht bei zu langsamer Geschwindigkeit ein lokales Verbrennen des Gewebes, und bei zu schnellem Ziehen ein unvollständiges Verschweissen, oder evtl. gar kein Verschweissen. Aus dem Stand der Technik ist aus NL 1 019 350 ein Gerät zum Fördern eines Katheters bekannt. Der Katheter wird zwischen zwei Rollen mit parallel angeordneten Achsen hindurchgeführt, welche gegengleich drehen, wobei die eine Rolle mittels Federkraft auf die andere gedrückt wird, sodass der Katheter dazwischen eingeklemmt wird. US Patentanmeldung US 2014/0296633 A1 offenbart ein Gerät für ein Endoskop. Das Endoskop wird ebenfalls zwischen zwei Rollen mit parallel angeordneten Achsen hindurchgeführt, welche gegengleich drehen, wobei die eine Rolle mittels Federkraft auf die andere gedrückt wird, sodass der Katheter dazwischen eingeklemmt wird. WO 00/18463 zeigt ein Gerät für die Förderung eines Katheters für bildgebende Zwecke. Auch dieses Gerät funktioniert auf der Basis, dass der Katheter zwischen zwei Rollen mit parallel angeordneten Achsen hindurchgeführt ist, welche gegengleich drehen. EP 1 442 720 zeigt einen Apparat für das Manövrieren von flexiblen Kathetern in kardiovaskulären Systemen. Auch dieser Katheter wird zwischen zwei Rollen bewegt. Die japanische Veröffentlichung JP 09000492 zeigt ein Gerät für das Einführen und Herausziehen eines Endoskopes, wobei dieses mittels zweier gegenläufiger Rollen bewegt wird. In allen diese Publikationen wird kein Wort über die Problematik verloren, dass diese Geräte in einem Operationsraum betrieben werden sollen und daher völlig steril sein müssen bzw. nach jedem Einsatz erneut sterilisiert werden müssen. Aus der NL 1 019 350 C2 ist ein Gerät zum gesteuerten Befördern eines Kabels in Form eines Katheters bekannt geworden. Allerdings ist das Gerät nicht auf hohen Temperaturen über 180° C sterilisierbar und daher nicht für das Herausziehen eines Schweiss-Katheters aus einer Vene geeignet, denn das Gerät muss hierzu in einem Operationssaal eingesetzt werden, wofür hohe Anforderungen an die Sterilität aller Geräte und Instrumente gestellt werden. Die WO 93/20876 A1 zeigt einen elektronischen Sensor für die Verschiebung eines Ketheters und WO 2012/037213 A1 A1 offenbart einen Antriebsmechanismus für ein Katheter-Robotersystem. IN beiden Fällen bestehen die Geräte zum Teil aus Materialien, welche die für eine zuverlässige Sterilisation nötigen hohen Temperaturen von mindestens 180° C nicht geeignet sind und somit nicht steril gemacht werden können.

Die Aufgabe dieser Erfindung ist es deshalb, ein Gerät zu schaffen, welches dem Chirurgen diese heikle und auch müheselige Arbeit abnimmt und ein kontinuierliches, gleichförmig langsames und motorisches Herausziehen eines solchen Schweiss-Katheters aus einer Vene ermöglicht. Dabei soll das Gerät den Schweiss-Katheter mit einstellbarer Geschwindigkeit und gleichbleibender Förderkraft transportieren können, vorzugsweise durch Ziehen, wobei aber das Gerät auch ein Stossen des Katheters oder auch eines ähnlichen Kabels ermöglichen soll. Das Gerät soll ausserdem sicherstellen, dass bei einem aus irgendeinem Grund auftretenden Stoppen der Förderung des Glasfaserkabels der Laser sofort abgeschaltet wird, damit keine lokalen Verbrennungen möglich sind. Schliesslich soll es das Gerät erlauben, die zurückgelegte Förderstrecke zu erfassen und auch jederzeit einen manuellen Stopp mit gleichzeitigem Abschalten des Lasers erlauben. Eine besondere Herausforderung für die Konstruktion eines solchen Gerätes besteht darin, dass es im Operationsaal unweigerlich in der Nähe des Körpers des Patienten eingesetzt werden muss und daher komplett steril sein muss, bzw. es muss von Anwendung zu Anwendung von Neuem sterilisierbar sein, das heisst alle äusseren Teile müssen eine Temperatur von bis zu 180°C, wie sie in einem Sterilisator bzw. Autoklaven auftreten, unbeschadet überstehen.

Diese Aufgabe wird gelöst von einem Gerät zum gesteuerten Befördern eines Kabels in Form eines Katheters mit Lichtleiter bzw. einer Glasfaser oder eines Elektrokabels oder eines kabelartigen Röhrchens, das ein Antriebsrad und ein daran radial anpressbares Anpressrad einschliesst, zwischen denen das zu befördernde Kabel einklemmbar ist, sodass es durch Antrieb des Antriebsrades von einer Antriebseinheit mit zugehörigem Steuergerät und elektronischer Steuerung förderbar ist, und die Drehbewegung und Drehgeschwindigkeit des Antriebsrades mittels einer Messeinrichtung messbar ist, wobei sich dieses Gerät dadurch auszeichnet, dass es dreiteilig ausgeführt ist, mit erstens einer auf mindestens 180°C sterilisierbaren Deckelplatte auf welcher aussen das Antriebsrad und das daran radial anpressbare Anpressrad gelagert ist, zweitens einer nicht sterilisierbaren Antriebseinheit mit elektronischer Steuerung, Elektromotor, Untersetzungsgetriebe und Energieversorgung, sowie drittens einem auf mindestens 180°C sterilisierbaren schachtelförmigen Gehäuse, in welches die Antriebseinheit vollständig einsetzbar ist und mit der Deckelplatte steril verschliessbar ist, sodass das Gerät von aussen allseits komplett steril ist, und einem zugehörigen Steuergerät, welches über ein elektrisches Kabel mit dem sterilisierten Gerät verbindbar ist,

Anhand der Zeichnungen wird eine beispielsweise Ausführung des Gerätes näher beschrieben und die Funktionen der einzelnen Bestandteile werden erklärt.

Es zeigt:
- Figur 1:: Das Gerät zum gesteuerten Befördern eines Katheters, Lichtleiters oder Kabels mit seinem zugehörigen Steuergerät und einem eingespannten Glasfaserkabel;
- Figur 2:: Die Deckelplatte des Gerätes mit dem Antriebs- und Messrad sowie den Anpressrädern von oben gesehen;
- Figur 3:: Die Unterseite der Deckelplatte mit den Transmissionsrädern und den Schlitten, in denen die Anpressräder in radialer Richtung gegen Federkraft vom Antriebs- bzw. Aufnahmerad verschiebbar sind;
- Figur 4:: Das Gerät nach Figur 1 in einer Explosionszeichnung von schräg oben gesehen, mit Deckelplatte, in das Gehäuse einsetzbarer Antriebseinheit und unten dem Gehäuse;
- Figur 5:: Das Gerät nach Figur 4 in einer Explosionszeichnung von schräg unten gesehen, mit Deckelplatte, in das Gehäuse einsetzbarer Antriebseinheit und unten dem Gehäuse;
- Figur 6:: Die obere Montageplatte der Antriebseinheit des Gerätes von oben gesehen;
- Figur 7:: Die Antriebseinheit des Gerätes von der Seite her gesehen;
- Figur 8:: Die obere Montageplatte der Antriebseinheit des Gerätes in umgedrehter Lage, auf ihre Unterseite in Perspektive gesehen;
- Figur 9:: Die auf das Gehäuse aufsetzbare Deckelplatte von unten gesehen, darunter die obere Montageplatte der Antriebseinheit und darunter das Gehäuse je von schräg unten gesehen;
- Fig. 10: Das zum Gerät gehörende Steuergerät mit nach oben abgehobenem Deckel mit den Steuertasten.

Bei einer Venenverödung schiebt der Arzt ein ca. 1.2 mm dickes Fiberglaskabel von unten nach oben in die Beinvene hinein. Wenn dieses richtig positioniert ist, wird ein Dioden-Laser mit einer Leistung von ca. 8 Watt und einer Wellenlänge von 1470nm eingeschaltet und bleibt ca. 3 Sekunden an Ort und Stelle brennen. Anschliessend wird das Fiberkabel mit einer gleichbleibenden Geschwindigkeit von etwa 1 mm/s mit aktiviertem Laser aus der Vene herausgezogen, damit die Vene verödet. Das Problem für den Arzt ist es nun, während einer Dauer von zum Beispiel 10 Minuten das Fiberkabel mit möglichst gleichförmiger Geschwindigkeit herauszuziehen. Bei einer Venenverödung wird die Laser-Leistung oder die Transportgeschwindigkeit in der Regel dem Venendurchmesser angepasst. Im oberen Beinbereich wird mehr Leistung zugeführt als im unteren Bereich. Daher wird der Vorgang in zum Beispiel drei verschiedene Verödungssequenzen unterteilt. Die einstellbare Laserleistung/Transportgeschwindigkeit ermitteln die Ärzte anhand einer einfachen Formel. Mittels eines voreinstellbaren Zeitgebers kann die Gesamtdauer der Verödungssequenzen eingestellt werden. Das vorliegende Gerät soll nun dieses Glasfaserkabel rein mechanisch mit gleichförmiger Geschwindigkeit aus der Vene herausziehen. Bei Unregelmässigkeiten oder nach Programmende soll ein akustisches Signal abgegeben werden.

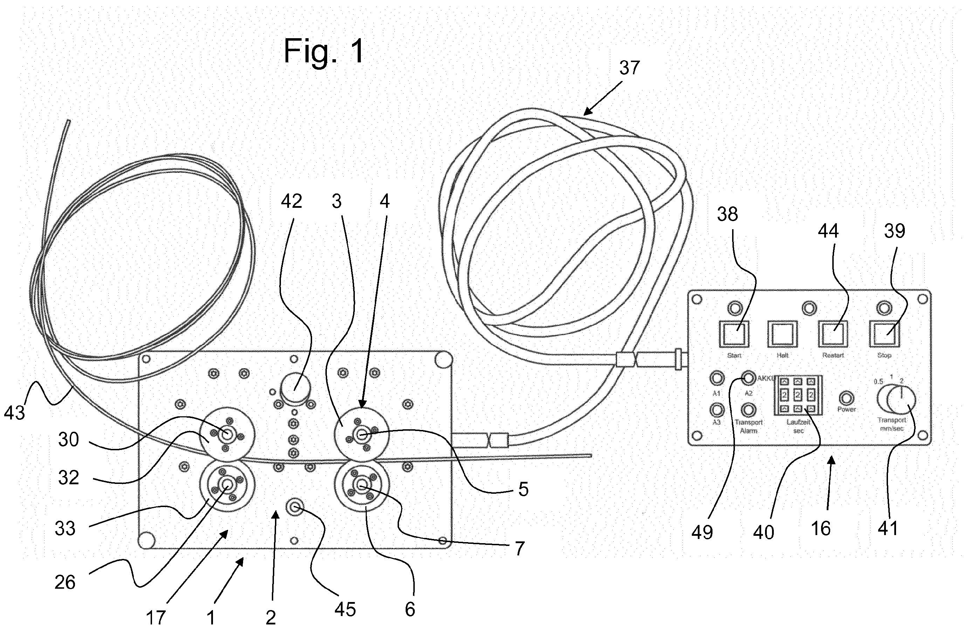

In Figur 1 ist ein solches Gerät mit seinem dazu gehörenden Steuergerät 16 von oben gesehen dargestellt. Das Gerät besteht aus einem Gehäuse 1, einer darin untergebrachten, herausnehmbaren Antriebseinheit und einer Deckelplatte 17, mit welcher das Gehäuse 1 von oben verschliessbar ist. Das Bild zeigt die Aussenseite 2 des Gerätes bzw. der Deckelplatte 17. Das zu fördernde Glasfaserkabel 43 führt durch zwei Paare von Rädern 32,33 und 3,6, die oben auf der Deckelplatte 17 angeordnet sind und deren Achsen bzw. Wellen 26,30; 5,7 durch die Deckelplatte 17 hindurch ins Innere des Gehäuses 1 führen. Zunächst wird das Glasfaserkabel 43 zwischen den zwei Rädern 32 und 33 hindurch geführt, welche zur Bestimmung der Fördergeschwindigkeit dienen, nämlich zwischen einem Messrad 32 mit aussen in seiner Umfangsfläche umlaufender Aufnahmenut für die Aufnahme des Glasfaserkabels 43 und einem zugehörigen Anpressrad 33, welches in diese Nut einpasst und welches von Hand gegen Federkraft mit seiner Achse bzw. Welle 26 in radialer Richtung von der Achse bzw. Welle 30 des Messrades 32 weg verschiebbar gelagert ist. Wenn die beiden Räder 32,33 so voneinander weg distanziert sind, kann das Faserkabel 43 zwischen sie eingelegt werden, und wenn das Anpressrad 33 wieder losgelassen wird, so presst es das Faserkabel 43 in die Nut in der Umfangsfläche des Messrades 32. In gleicher Weise lässt sich die Welle 7 des im Bild weiter rechts sichtbaren Anpressrades 6 von Hand in radialer Richtung in Bezug auf die Welle 5 des Antriebsrades 3 von dessen Welle 5 weg verschieben, um zwischen den beiden Rädern 3,6 das Einlegen des Glasfaserkabels 43 zu erlauben. Hernach wird die Achse bzw. Welle 7 des Anpressrades 6 losgelassen und es wird fortan mit Federkraft auf die Umfangsfläche des Antriebsrades 3 gepresst, sodass sich das dazwischen eingeklemmte Faserkabel 43 vom Antriebsrad 3 fördern lässt. Das Antriebsrad 3 kann hierzu eine Umfangsfläche 4 mit einer darin eingelassenen Nut mit gerändeltem Nutboden aufweisen, um die Traktion zu verbessern. Der Drehknopf 42 auf der Deckelplatte 17 dient als Hauptschalter für die Antriebseinheit unterhalb dieser Deckelplatte 17, und mit 45 ist eine Bereitschaftsleuchte gezeigt. Ein vieladriges Elektrokabel 37 führt zum zugehörigen Steuergerät 16 mit seinen verschiedenen Organen. Auf dessen Oberseite erkennt man eine Starttaste 38, eine Stopptaste 39 und eine Re-Start Taste 44. Mit 40 ist ein Zählwerk mit Reset-Taste bezeichnet, welches die jeweils zurückgelegte Förderstrecke des Faserkabels 43 gemäss Messung durch das Messrad 32 angibt. Mit dem Drehknopf 41 lässt sich die Fördergeschwindigkeit regulieren bzw. einstellen.

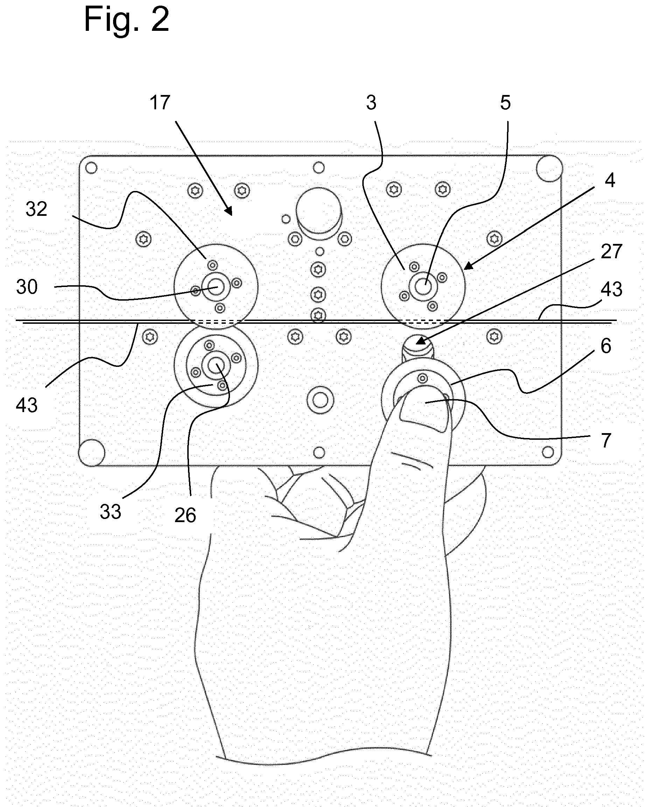

Die Figur 2 zeigt die Deckelplatte 17 des Gerätes in gesonderter und vergrösserter Darstellung. Zu erkennen sind links das Messrad 32 mit seiner Welle 30 und das Anpressrad 33 mit seiner Welle 26 für dieses Messrad 32. Das Anpressrad 33 lässt sich gegen eine Federkraft in radialer Richtung vom Messrad 32 weg verschieben, wobei die Achsen bzw. Wellen 30,26 der beiden Räder 32,33 stets parallel zueinander verlaufend bleiben. Der Verschiebeweg ist so lang bemessen, dass eine Lücke zwischen den Rädern 32,33 entstehen kann, sodass das Glasfaserkabel 43 zwischen diese Lücke in die in der Umfangsfläche des Messrades 32 verlaufende Nut gelegt werden kann. Hernach wird das Anpressrad 33 wieder entlastet und drückt hernach mit seiner Federkraft auf das in der Nut liegende Glasfaserkabel. In gleicher Weise kann das Anpressrad 6 im Bild weiter rechts vom Antriebsrad 3 gegen eine Federkraft mit einem Finger weg verschoben werden, wie im Bild gezeigt. Seine Welle 7 ist in einem verschiebbaren Schlitten gelagert und kann längs der Ausnehmung 27 in der Deckelplatte 17 verschoben werden. Bei einer Verschiebung wie gezeigt kann das Glasfaserkabel zwischen den beiden Rädern 3,6 in die Nut in der Umfangsfläche 4 des Antriebsrades 3 gelegt werden und hernach presst das Anpressrad 6 das Glasfaserkabel aufgrund der wirkenden Federkraft in diese Nut. Das Antriebsrad 6 kann in seiner Nut mit einer Rändelung versehen sein, um die Traktion zum Glasfaserkabel zu verbessern.

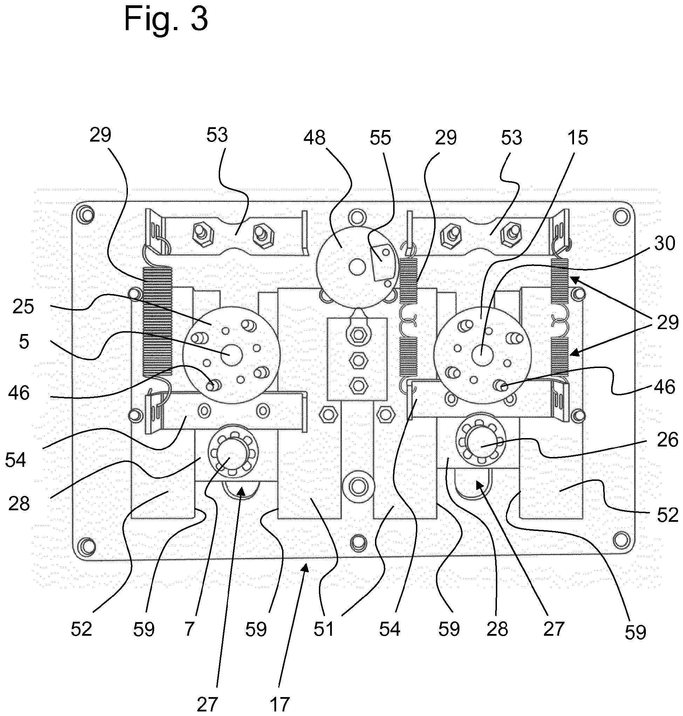

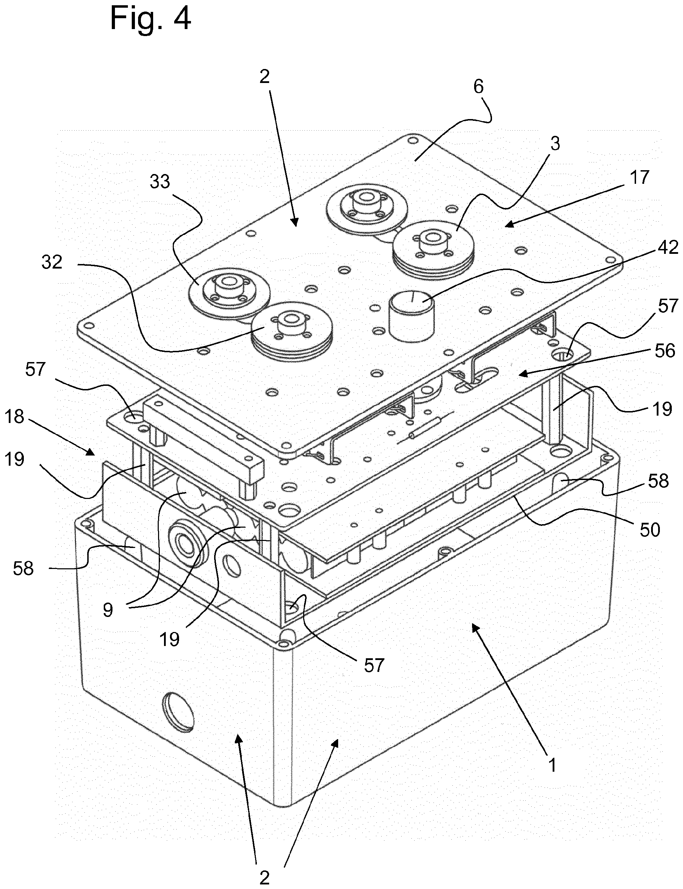

Die Figur 3 zeigt diese Deckelplatte 17 auf ihre Unterseite gesehen. Die Welle 5 des Antriebsrades, in der linken Bildhälfte, trägt auf dieser Seite ein fest mit der Welle verbundenes Transmissionsrad 25 mit axial abstehenden Nocken 46, wobei die Welle 5 stationär in der Deckelplatte 17 gelagert ist. Die Welle 7 des Anpressrades 6 hingegen durchsetzt die Deckelplatte 17 in einer Ausnehmung 27 und ist in einem Schlitten 28 gelagert, welcher auf dieser hier sichtbaren Unterseite der Deckelplatte 17 in zwei einander gegenüberliegenden Schlitzen 59 verschiebbar ist, welche durch die beiden Führungsplatten 51,52 gebildet sind, die mit etwas Abstand zur Oberfläche der Deckelplatte 17 auf dieser angebracht sind. Der Schlitten 28 ist mit einer Halterungen 54 verbunden, welche auf beiden Seiten der Welle 7 mit dem Ende von zwei Zugfedern 29 verbunden ist, wobei die anderen Enden der Zugfedern 29 an einer stationären Halterung 53 an der Unterseite der Deckelplatte 17 befestigt sind. Somit lässt sich die Achse bzw. Welle 7 des Anpressrades 6 von der Welle 5 des Antriebsrades 3 gegen die Kraft der beiden Zugfedern 29 mit einem Finger wegverschieben, wie gezeigt. Die Welle 26 des Anpressrades 33 für das Messrad in der rechten Bildhälfte ist in genau gleicher Weise verschiebbar in einem Schlitten 28 gelagert und gegen die Kraft von Zugfedern 29 verschiebbar. Unterhalb des Messrades ist das Treibrad 15 mit seinen Nocken 46 zu erkennen, welches seine Kraft auf ein Rad an der Antriebseinheit 18 (Figur 4) weitergibt. Die zum Drehknopf 42 gehörende Achse trägt auf der Unterseite der Deckelplatte 17 eine Scheibe 55 mit einem Kontaktelement 48, um die Antriebseinheit 18 ein- und auszuschalten.

In Figur 4 ist das Gerät in einer Explosionszeichnung von schräg oben gesehen dargestellt, mit Deckelplatte 17, in das Gehäuse 1 einsetzbarer Antriebseinheit 18 und unten dem Gehäuse 1. Die Antriebseinheit 18 ist auf einer Grundplatte 50 angeordnet, auf welcher die Batterien 9 für einen Elektromotor untergebracht sind, sowie die nötigen elektronischen Steuerungskomponenten für den Elektromotor und die Bewirtschaftung der Energieversorgung. Auf der Unterseite einer weiteren, oben auf Distanzstützen 19 angeordneten Montageplatte 56 der Antriebseinheit 18 befindet sich der Elektromotor, seine Antriebsachse mit anschliessendem Untersetzungsgetriebe und die elektronischen Komponenten für die Umsetzung der Drehung des Messrades 32 in ein elektrisches Signal. Auf der Oberseite der Montageplatte 56 sind weitere Komponenten untergebracht, die in einer gesonderen Ansicht noch gezeigt und beschrieben werden. Diese gesamte Antriebseinheit 18 lässt sich im Gehäuse 1 versenken. Hierzu dienen hier Führungsdorne 58 auf dem Gehäuseboden, über welche die untere Grundplatte 50 und die Montageplatte 56 mit ihren Löchern 57 stülpbar ist, und hernach kommt die Deckelplatte 17 oben auf das Gehäuse 1 und wird mit ihm an ihren Ecken verschraubt, unter Einschluss der gesamten Antriebseinheit 18. Die Deckelplatte 17 mit ihren mechanischen Komponenten sowie auch das leere Gehäuse 1 lassen sich für jede neue Anwendung zuvor sterilisieren, indem sie in einem Sterilisiergerät auf 180°C erhitzt werden. Sie können dabei keinen Schaden nehmen, weil sei keinelei temperatursensible Teile beinhalten. Hernach wird die unsterile und wegen ihrer temperatursensitiven elektronischen und elektrischen Komponenten nicht sterilisierbare Antriebseinheit 18 in das Gehäuse 1 hineingelegt und mit der daraufgelegten Deckelplatte 17 fest verschraubt, wonach das Gerät in seiner Gesamtheit steril ist, weil alle äusseren Flächen 2 und Komponenten sterilisiert sind.

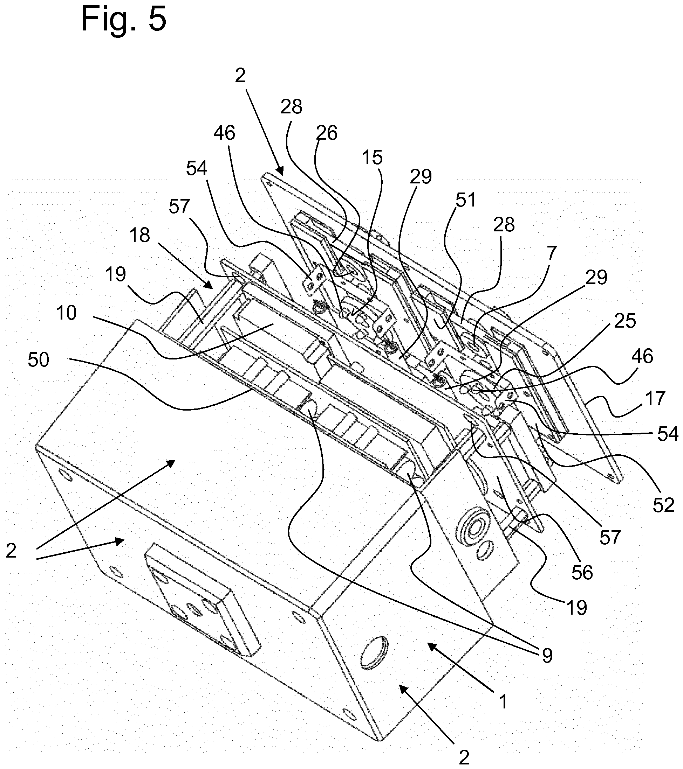

Die Figur 5 zeigt das Gerät nach Figur 4 in einer Explosionszeichnung von schräg unten gesehen, mit Deckelplatte 17, in das Gehäuse 1 einsetzbarer Antriebseinheit 18 und unten dem Gehäuse 1. Auf der Unterseite der Deckelplatte 17 erkennt man die Transmissionsräder 25 mit ihren nach unten abstehenden Nocken 46. Anhand einer weiteren Zeichnung wird klar, wie die Antriebskraft aus der Antriebseinheit 18 auf das Transmissionsrad 25 übertragen wird, und in umgekehrte Richtung die Drehung des Messrades 32 über das Treibrad 15 auf das Zählrad in der Antriebseinheit 18. Diese Lösung ermöglicht den höchst einfachen Ausbau und wieder Einbau der Antriebseinheit 18 für die Zwecke der Sterilisierung. Das leere Gehäuse 1 und die Deckelplatte 17 können mit ihren rein mechanischen Komponenten vor jedem Einsatz sterilisiert werden und hernach kann die Antriebseinheit 18 einfach wieder ins Gehäuse 1 gesteckt und die Deckelplatte 17 darauf geschraubt werden.

Hier in Figur 5 sieht man auf der Unterseite der Deckelplatte 17 die Führungsplatten 51,52 für die Halterung und Führung des Schlittens 28, in welchem die verschiebbare Welle 7 für das Anpressrad gelagert ist. Der Schlitten 28 trägt auch die mit ihm verschiebbare Halterung 54 für die Zugfedern 29 für die Rückführung der Verschiebung nach dem Loslassen des Anpressrades und seiner Welle 7. In genau gleicher Weise ist die Verschiebbarkeit der Welle 26 des Anpressrades für das Messrad gelöst, wie man links daneben im Bild erkennt. Unterhalb des Messrades treibt dieses ein Treibrad 15 mit seinen Nocken 46 an.

Unterhalb der Deckelplatte 17 erkennt man die Antriebseinheit 18. Unterhalb der Montageplatte 56 ist die Elektronik 10 für die Steuerung des Elektromotors und für die Bewirtschaftung der Energieversorgung untergebracht, welche aus einem Paket von Batterien 9 besteht. Diese ganze Antriebseinheit 18 wird in weiteren Bildern noch näher vorgestellt und beschrieben. Sie lässt sich als gesamte Einheit in das Gehäuse 1 absenken, indem sie mit ihren Löchern 57 an den Ecken in ihrer hier nicht sichtbaren Grundplatte und in der Montageplatte 56 über Führungsdorne im Gehäuse 1 gestülpt werden kann. Hernach kann die Deckelplatte 17 unter komplettem Einschluss der Antriebseinheit 18 auf das Gehäuse 1 aufgeschraubt werden und das ganze Gerät, das heisst alle seine nach aussen gewandten Teile 2 und Wände sind dann steril.

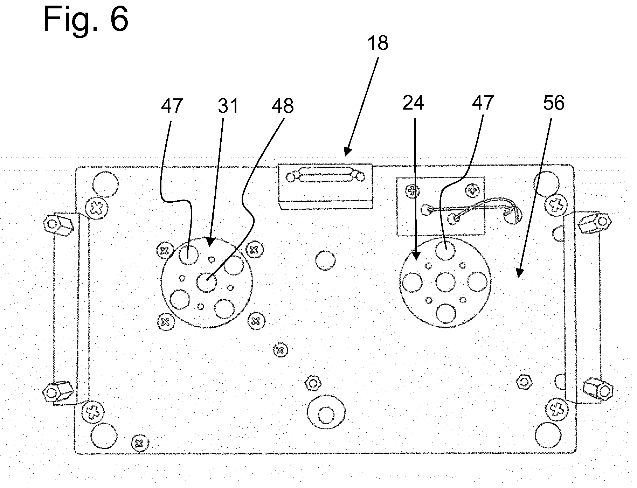

Die Figur 6 zeigt die Oberseite der Montageplatte 56 der Antriebseinheit 18. Das Rad auf der rechten Bildseite ist das Treibrad 24, welches über ein Getriebe vom Elektromotor unterhalb der Montageplatte 56 angetrieben wird. Es dient zur Übertragung der Antriebskraft auf das Transmissionsrad 25 auf der Unterseite der Deckelplatte 17. Das Aufnahmerad 31 in der linken Hälfte des Bildes hingegen dient in umgekehrter Richtung zur Übertragung der Antriebskraft, die vom Messrad und seinem Treibrad 15 herrührt. Das Messrad oben auf der Deckelplatte 17 wird vom Glasfaserkabel angetrieben, wenn dieses vom Antriebsrad 3 angetrieben wird und somit zwischen dem Messrad 32 und seinem Anpressrad 33 hindurch gezogen wird. Das Treibrad 15 des Messrades 32 gibt seine Antriebskraft weiter nach unten auf dieses hier gezeigte Aufnahmerad 31, indem die Nocken 46 am Treibrad 15 in die Löcher 47 im Aufnahmerad 31 drehmomentschlüssig eingreifen. Das Aufnahmerad 31 gibt die Drehung weiter an eine Zählscheibe 12 unterhalb der Montageplatte 56. Jetzt ist klar, wie die Unterseite der Deckelplatte 17 auf diese Oberseite der Montageplatte 56 aufsetzbar ist, nämlich so, dass die Nocken 46 an den Rädern auf der Unterseite der Deckelplatte 17 in die Löcher 47 an den Rädern 24,31 auf der Oberseite der Montageplatte 56 drehmomentschlüssig eingreifen, nämlich des Treibrades 15 und des Aufnahmerades 31 des Messrades.

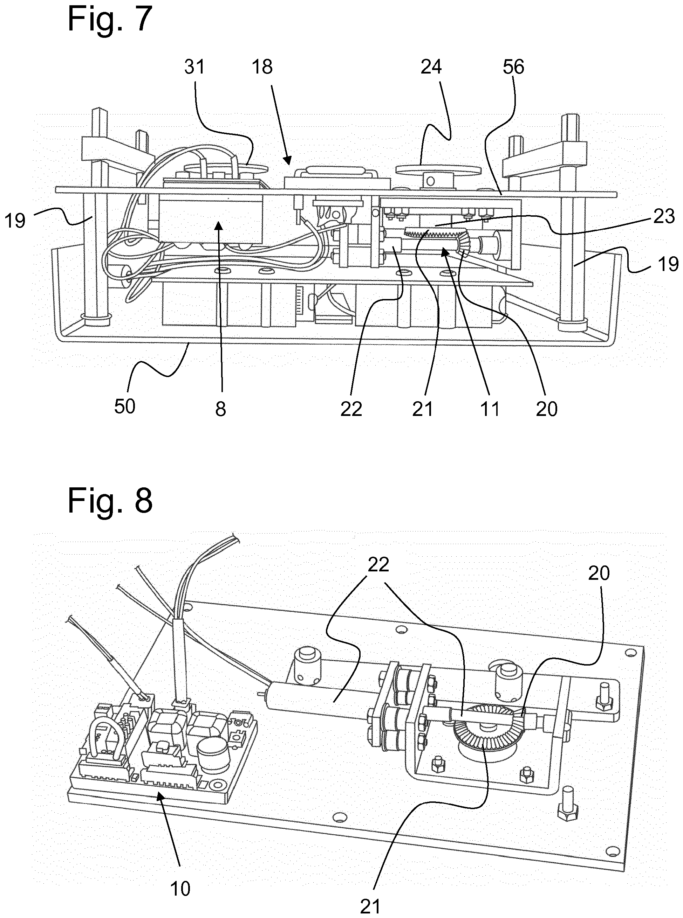

Die Figur 7 zeigt die gesamte Antriebseinheit 18 in einer Ansicht von der Seite her gesehen, mit oben der Montageplatte 56 und unten der Grundplatte 50. Unten an der Montageplatte 56 sind der Elektromotor 8 seine Abtriebswelle 22 und ein Kegelzahnrad 20 an ihrem Ende verbaut. Dieses Kegelzahnrad 20 greift in ein Zahnrad 21 ein, das mit der Welle 23 dreht, auf welcher das Treibrad 24 oberhalb der Montagplatte 56 sitzt. Damit wird ein Untersetzungsgetriebe 11 mit starker Untersetzung gebildet, sodass die Welle 23 sehr langsam und mit kräftigem Drehmoment drehen kann. Das am Treibrad 24 anfallende Drehmoment wird über die eingreifenden Nocken 46 des Transmissionsrades 24 schliesslich an das Antriebsrad 3 weitergegeben. In umgekehrter Richtung fliesst die Kraft vom Messrad hinunter zum Aufnahmerad 31 und dieses treibt eine Zählscheibe an, wie das noch gezeigt wird.

Die Figur 8 zeigt die Unterseite der Montageplatte 56 mit der Abtriebswelle 22 des hier nicht gezeigten Elektromotors, sowie das Kegelzahnrad 20 und das von ihm angetriebene Zahnrad 21, dessen Welle letztlich das Antriebsrad 24 aussen an der Antriebseinheit 18 antreibt. Ebenfalls an der Montageplatte 56 angebaut ist die elektronische Steuerung 10 mit ihren Komponenten.

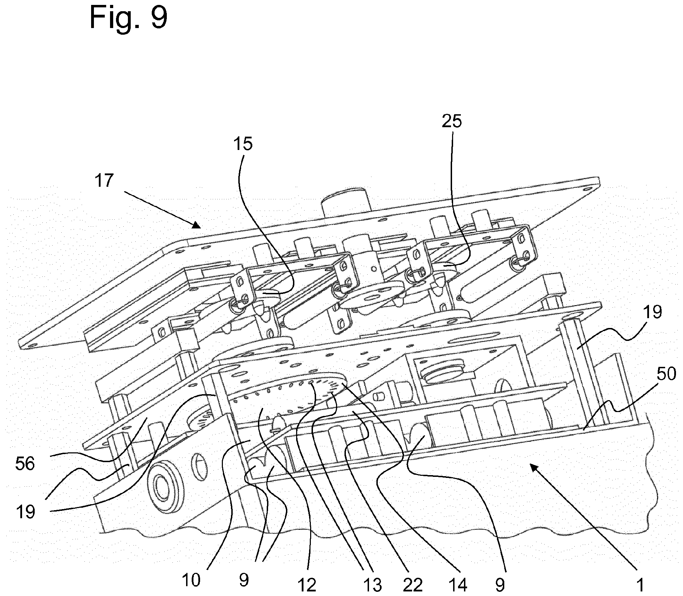

Aus der Figur 9 erschliesst sich, wie die Messung der Geschwindigkeit der Förderung realisiert ist. Unten an der Montageplatte 56 dreht eine Zählscheibe 12 mit der vom Messrad stammenden Drehung mit. Die Zählscheibe 12 weist in ihrem Umfangsbereich 14 radiale Schlitze 13 oder Löcher auf. Senkrecht zur Zählscheibe 12 ist eine Lichtschranke eingerichtet, deren Lichtstrahl durch den Lochbereich der Zählscheibe 12 führt und jenseits auf einen Lichtsensor auftrifft. Durch die Anzahl eintreffender Lichtpulse pro Zeit lässt sich von der zugehörigen Elektronik die Fördergeschwindigkeit des Glasfaserkabels ermitteln. Das entsprechende elektrische Signal wird über das Kabel 37 der Steuerungseinheit 16 zugeführt (Figur 1). Sollte die Geschwindigkeit durch eine Blockierung Null werden, so unterbricht dieses Steuergerät 16 augenblicklich die Stromzufuhr zum Laser, um Verbrennungen in der Vene zu vermeiden. Wenn eine zuvor gewählte Förderstrecke fertig abgefahren ist, stoppt die Förderung und das Steuergerät 16 erzeugt einen Signalton.

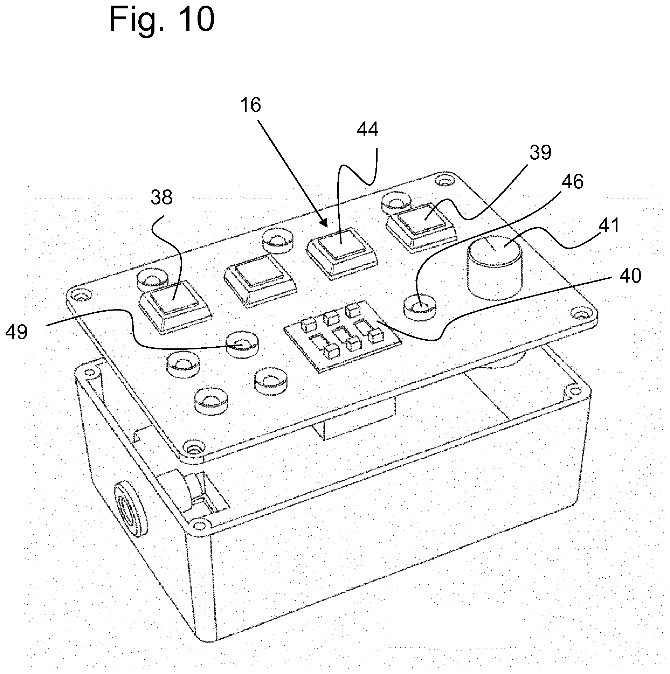

Die Figur 10 zeigt das gesonderte Steuergerät 16, welches einen oder mehrere Meter vom eigentlichen Gerät für die Förderung des Glasfaserkabels entfernt aufgestellt sein kann und daher nicht notwendigerweise steril sein muss und wegen seinen elektronischen Komponenten auch nicht sterilisierbar ist. Sonst müsste es in einen sterilen Plastikbehälter untergebracht werden, sodass seine Tasten von aussen durch den sterilisierbaren Plastikbehälter hindurch betätigbar wären. Mit diesem Steuergerät 16 sind mittels der elektronischen Steuerung 10 in der Antriebseinheit 18 wenigstens folgende Funktionen schaltbar und umsetzbar:
- Ein- und Ausschalten des Gerätes zur Verbindung oder Trennung von der Energieversorgung und Einschalten des Lasers für die Zählscheibe 21
- Drehknopf 41 für stufenlose oder diskrete Wahl der Fördergeschwindigkeit des Kabels 43
- Anzeige der gewählten Fördergeschwindigkeit des Glasfaserkabels am Steuergerät 16
- Wahl einer abzufahrenden Förderstrecke
- Bereitschaftsleuchte, zeigt Gerät ist bereit zum Start der Förderung
- Starttaste 38 zum Starten der Förderung
- Stopptaste 39 zum jederzeitigen Stoppen der Förderung
- Anzeige 40 der jeweils zurückgelegten Förderstrecke
- Restart-Taste 44 zum Nullstellen des angezeigten Förderweges
- Anzeige 49 der Batterieladung bei einer Energieversorgung mittels Batterien
- Automatisches Abstellen des Katheterlasers bei Stopp der Förderung. Hierzu führt ein nicht eingezeichnetes Steuerkabel zum Generator für den Laser
- Abgabe eines Signaltones bei fertig abgefahrener Förderstrecke.

### Ziffernverzeichnis

- 1: Gehäuse
- 2: Aussenseite des Gerätes
- 3: Antriebsrad
- 4: Umfangsfläche Antriebsrad 3
- 5: Welle des Antriebsrades 3
- 6: Anpressrad
- 7: Welle des Anpressrades 6
- 8: Elektromotor
- 9: Energieversorgung Elektromotor 8
- 10: elektronische Steuerung
- 11: Untersetzungsgetriebe für Antriebsrad 3
- 12: Zählscheibe
- 13: radiale Schlitze im Zählrad 12
- 14: Umfangsbereich der Zählscheibe
- 15: Treibrad des Messrades 32
- 16: Steuergerät
- 17: Deckelplatte
- 18: Antriebseinheit
- 19: Distanzstützen an der Antriebseinheit
- 20: Kegelzahnrad
- 21: Zahnrad
- 22: Welle des Antriebsrades am E-Motor 8
- 23: Welle Antriebsrad an der Antriebseinheit 18
- 24: Treibrad aussen an der Antriebseinheit 18
- 25: Transmissionsrad
- 26: Welle des Anpressrades 33
- 27: Ausnehmung in der Deckelplatte für die Verschiebung des Anpressrades
- 28: Schlitten an der Unterseite der Deckelplatte
- 29: Federn für das Anpressrad
- 30: Welle des Messrades 32
- 31: Aufnahmerad
- 32: Messrad aussen an der Deckelplatte
- 33: Anpressrad für das Messrad 32
- 34: Treibrad
- 35: Aufnahmerad für das Treibrad 34
- 36: Welle des Aufnahmerades 35
- 37: Elektrokabel
- 38: Starttaste
- 39: Stopptaste
- 40: Anzeige der zurückgelegten Förderstrecke
- 41: Drehknopf für Regulierung Fördergeschwindigkeit
- 42: Drehknopf als Hauptschalter Energieversorgung
- 43: zu förderndes Kabel
- 44: Restart-Taste
- 45: Bereitschaftsleuchte
- 46: Nocken
- 47: Ausnehmungen für Noppen
- 48: Kontaktelement auf Scheibe 55
- 49: Anzeige Batterieladung
- 50: Grundplatte Antriebseinheit 18
- 51: Führungsplatte für Schlitten 28
- 52: Führungsplatte für Schlitten 28
- 53: stationäre Halterung für Federn 29
- 54: verschiebbare Halterung auf dem Schlitten für die Federn 29
- 55: Scheibe unten am Drehknopf
- 56: Montageplatte
- 57: Führungslöcher
- 58: Führungsdorne auf Gehäuseboden
- 59: Schlitze für den Schlitten

## Patentansprüche

1. Gerät zum gesteuerten Befördern eines Kabels in Form eines Katheters mit Lichtleiter bzw. einer Glasfaser oder eines Elektrokabels oder eines kabelartigen Röhrchens, das ein Antriebsrad (3) und ein daran radial anpressbares Anpressrad (6) einschliesst, zwischen denen das zu befördernde Kabel (43) einklemmbar ist, sodass es durch Antrieb des Antriebsrades (3) von einer Antriebseinheit (18) mit zugehörigem Steuergerät (16) und elektronischer Steuerung förderbar ist, und die Drehbewegung und Drehgeschwindigkeit des Antriebsrades (3) mittels einer Messeinrichtung messbar ist
***dadurch gekennzeichnet*,**
**dass** das Gerät dreiteilig ausgeführt ist, mit erstens einer auf mindestens 180°C sterilisierbaren Deckelplatte (17) auf welcher aussen das Antriebsrad (3) und das daran radial anpressbare Anpressrad (6) gelagert ist, zweitens einer nicht sterilisierbaren Antriebseinheit (18) mit elektronischer Steuerung (10), Elektromotor (8), Untersetzungsgetriebe (11) und Energieversorgung (9), sowie drittens einem auf mindestens 180°C sterilisierbaren schachtelförmigen Gehäuse (1), in welches die Antriebseinheit (18) vollständig einsetzbar ist und mit der Deckelplatte (17) steril verschliessbar ist, sodass das Gerät von aussen allseits komplett steril ist, und einem zugehörigen Steuergerät (16), welches über ein elektrisches Kabel (37) mit dem sterilisierten Gerät verbindbar ist.

2. Gerät nach Anspruch 1, ***dadurch gekennzeichnet,* dass** die Drehbewegung des Antriebsrades (3) auf eine synchron zu ihm laufende Zählscheibe (12) mit radialen Schlitzen (13) oder Löchern im Umfangsbereich übersetzt ist, die durch eine stationäre Lichtschranke drehbar ist, zur Bestimmung der Bewegung und Drehgeschwindigkeit des Antriebsrades (3).

3. Gerät nach einem der vorangehenden Ansprüche, ***dadurch gekennzeichnet,* dass** das Gehäuse (1) auf seiner Aussenseite (2) ein Antriebsrad (3) aufweist, mit einem dazu in radialer Richtung auf seine Umfangsfläche (4) beaufschlagenden, rechtwinklig zu seiner Achse (5) verschiebbaren Anpressrad (6) mit parallel zu jener des Antriebsrades (3) gelagerten Achse (7), sodass zwischen Antriebsrad (3) und Anpressrad (6) das Kabel (43) zwecks Förderung einklemmbar ist, und dass die Antriebseinheit (18) im Innern des Gehäuses (1) einen Elektromotor (8) mit zugehöriger Energieversorgung (9) und elektronischer Steuerung (10) aufweist, von welchem das Antriebsrad (3) über ein Untersetzungsgetriebe (11) antreibbar ist, und dass innerhalb des Gehäuses (1) eine Zählscheibe (12) mit radialen Schlitzen (13) oder Löchern in ihrem Umfangsbereich (14) vorhanden ist, welche von einer stationären Lichtschranke durchquerbar sind, zur Erfassung von Bewegung und Rotationsgeschwindigkeit der Zählscheibe (12) des Messrades (32) und somit des Antriebsrades (3), zur Generierung von elektrischen Signalen für die Verarbeitung und Anzeige an einem mittels eines Elektrokabels (37) verbindbaren zugehörigen Steuergerät (16), von welchem aus das ganze Gerät steuerbar ist.

4. Gerät nach einem der vorangehenden Ansprüche, ***dadurch gekennzeichnet*, dass** der Elektromotor (8) innerhalb der Antriebseinheit (18) mit seiner Abtriebsachse mit einer Welle (22) verbunden ist, die ein Kegelzahnrad (20) trägt, welches in Eingriff mit einem Zahnrad (21) auf der Welle (23) des aussen an der Antriebseinheit (18) befindlichen Treibrades (24) steht, welches seinerseits drehmomentschlüssig mit einem Transmissionsrad (25) an der Unterseite der Deckelplatte (17) in Eingriff bringbar ist, wobei dieses Transmissionsrad (25) auf der Welle (5) des aussen an der Deckelplatte (17) befindlichen Antriebsrades (3) sitzt.

5. Gerät nach einem der vorangehenden Ansprüche, ***dadurch gekennzeichnet*, dass** das zum Antriebsrad (3) aussen an der Deckelplatte (17) gehörige verschiebbare Anpressrad (6) eine Welle (7) aufweist, welche die Deckelplatte (17) in einer Ausnehmung (27) durchsetzt, und diese Welle (7) an einem Schlitten (28) an der Unterseite der Deckelplatte (17) gegen die Kraft von Federn (29) vom Antriebsrad (3) weg verschiebbar geführt ist.

6. Gerät nach einem der Ansprüche 4 bis 5, ***dadurch gekennzeichnet*, dass** das Antriebsrad (3) aussen an der Deckelplatte (17) mit seiner Welle (5) an der Unterseite der Deckelplatte (17) ein Transmissionsrad (25) trägt, zum drehmomentschlüssigen Koppeln mit dem Treibrad (24) oben auf der Antriebseinheit (18) beim Aufsetzen der Deckelplatte (17) auf das Gehäuse (1).

7. Gerät nach einem der vorangehenden Ansprüche, ***dadurch gekennzeichnet*, dass** an der Deckelplatte (17) ein frei drehendes Messrad (32) mit einer Welle (30) parallel zur Welle (5) des Antriebsrades (3) gelagert ist, mit eigenem federbelasteten Anpressrad (33) wie jenes Anpressrad (6) zum Antriebsrad (3), und dass die Welle (30) des Messrades (32) auf der Unterseite der Deckelplatte (17) ein Treibrad (15) für ein Aufnahmerad (31) an der Oberseite der Antriebseinheit (18) trägt, mit welchem es beim Aufsetzen der Deckelplatte (17) auf das Gehäuse (1) drehmomentschlüssig koppelbar ist, und welches Aufnahmerad (31) mit seiner Welle im Innern der Antriebseinheit (18) die Zählscheibe (12) trägt.

8. Gerät nach einem der vorangehenden Ansprüche, ***dadurch gekennzeichnet*, dass** das Gerät und seine elektronische Steuerung (10) über ein Elektrokabel (37) durch das entfernte Steuergerät (16) bedienbar ist.

9. Gerät nach einem der Ansprüche 4 bis 8, ***dadurch gekennzeichnet*, dass** die drehmomentschlüssige Verbindung vom Treibrad (24) zum Transmissionsrad (25) durch ein axiales Ineinandergreifen derselben realisiert ist, genauso wie auch die drehmomentschlüssige Verbindung des Treibrades (15) auf der Unterseite der Deckelplatte (17) zum Aufnahmerad (31) an der Oberseite der Antriebseinheit (18), welches mit seiner Welle (48) im Innern der Antriebseinheit (18) die Zählscheibe (12) trägt.

10. Gerät nach Anspruch 9, ***dadurch gekennzeichnet*, dass** die drehmomentschlüssige Verbindung vom Treibrad (24) zum Transmissionsrad (25) und die drehmomentschlüssige Verbindung vom Treibrad (15) auf das Aufnahmerad (31) realisiert sind, indem das jeweils eine Rad axiale Nocken (46) aufweist, welche bei auf das Gehäuse (1) aufgesetzter Deckelplatte (17) in Ausnehmungen (47) im anschliessenden Rad (25,31) eingreifen.

11. Gerät nach einem der vorangegangenen Ansprüche, ***dadurch gekennzeichnet*, dass** die Energieversorgung (9) durch eine Anzahl wieder aufladbarer Batterien im Innern der Antriebseinheit (18) sichergestellt ist, welche über das Elektrokabel (37) aufladbar sind.

12. Gerät nach einem der vorangehenden Ansprüche, ***dadurch gekennzeichnet*, dass** mit dem Steuergerät (16) und der mit ihr verbundenen elektronischen Steuerung (10) in der Antriebseinheit (18) wenigstens folgende Funktionen schaltbar und umsetzbar sind:
• Ein- und Ausschalten des Gerätes zur Verbindung oder Trennung von der Energieversorgung und Einschalten des Lasers für die Zählscheibe (12)
• Drehknopf (41) für stufenlose oder diskrete Wahl der Fördergeschwindigkeit des Kabels (43)
• Wahl der abzufahrenden Förderstrecke
• Bereitschaftsleuchte (46) für das Starten des Gerätes
• Anzeige der gewählten Fördergeschwindigkeit am Steuergerät (16)
• Starttaste (38) zum Starten der Förderung
• Stopptaste (39) zum Stoppen der Förderung
• Anzeige (40) der zurückgelegten Förderstrecke
• Restart-Taste (44) zum Nullstellen des angezeigten Förderweges
• Anzeige (49) der Batterieladung bei Energieversorgung mittels Batterien
• Automatisches Abstellen des Katheterlasers bei Stopp der Förderung via Steuerkabel an den Generator des Lasers
• Stoppen der Förderung, wenn die gewählte Förderstrecke abgefahren ist und Abgabe eines Signaltones.

## Claims

1. Apparatus for the controlled transport of a cable in the form of a catheter with a light conductor or an optical fibre respectively or an electric cable or a cable-like tube, which includes a drive wheel (3) and a pressure wheel (6) which can be pressed radially thereon, between which the cable (43) to be conveyed can be clamped, so that it can be conveyed by driving the drive wheel (3) by a drive unit (18) with associated control unit (16) and electronic control, and the rotary movement and rotary speed of the drive wheel (3) can be measured by means of a measuring device
***characterized by** this*,
in that the device is constructed in three parts, with firstly a cover plate (17) which can be sterilised to at least 180°C and on which the drive wheel (3) and the pressure wheel (6) which can be pressed radially against it are mounted externally, and secondly a non-sterilisable drive unit (18) with electronic control (10), electric motor (8), reduction gear (11) and power supply (9), and thirdly a box-shaped housing (1) which can be sterilized to at least 180°C, into which the drive unit (18) can be completely inserted and can be sterilely closed with the cover plate (17) so that the device is completely sterile on all sides from the outside, and an associated control unit (16) which can be connected to the sterilized device via an electrical cable (37).

2. Apparatus according to claim 1, ***characterized in that*** the rotational movement of the drive wheel (3) is transmitted to a counting disc (12) running synchronously therewith and having radial slots (13) or holes in the peripheral region, which is rotatable by a stationary light barrier, for determining the movement and rotational speed of the drive wheel (3).

3. Apparatus according to one of the preceding claims, ***characterised in that*** the housing (1) has a drive wheel (3) on its outside (2), with a pressure wheel (6) which acts on its circumferential surface (4) in the radial direction, is displaceable at right angles to its axis (5) and has an axis (7) mounted parallel to that of the drive wheel (3), so that the cable (43) can be clamped between the drive wheel (3) and the pressure wheel (6) for the purpose of conveying, and that the drive unit (18) has an electric motor (8) with associated power supply (9) and electronic control (10) inside the housing (1), by which the drive wheel (3) can be driven via a reduction gear (11), and that within the housing (1) there is a counting disc (12) with radial slots (13) or holes in its peripheral region (14), which can be traversed by a stationary light barrier, for detecting the movement and rotational speed of the counting disc (12) of the measuring wheel (32) and thus of the drive wheel (3), for generating electrical signals for processing and display on an associated control unit (16) which can be connected by means of an electrical cable (37) and from which the entire device can be controlled.

4. Apparatus according to any of the preceding claims, ***characterized in that*** the electric motor (8) inside the drive unit (18) is connected by its output shaft to a shaft (22) carrying a bevel gear (20) which is in mesh with a gear (21) on the shaft (23) of the driving wheel (24) located outside the drive unit (18), which in turn can be brought into torque-locking engagement with a transmission wheel (25) on the underside of the cover plate (17), this transmission wheel (25) being seated on the shaft (5) of the drive wheel (3) located on the outside of the cover plate (17).

5. Apparatus according to one of the preceding claims, ***characterised in that*** the displaceable pressure wheel (6) belonging to the drive wheel (3) externally on the cover plate (17) has a shaft (7) which passes through the cover plate (17) in a recess (27), and this shaft (7) is guided on a slide (28) on the underside of the cover plate (17) so as to be displaceable away from the drive wheel (3) against the force of springs (29).

6. Apparatus according to one of claims 4 to 5, ***characterised in that*** the drive wheel (3) carries a transmission wheel (25) externally on the cover plate (17) with its shaft (5) on the underside of the cover plate (17), for torque-locking coupling with the drive wheel (24) on top of the drive unit (18) when the cover plate (17) is placed on the housing (1).

7. Apparatus according to one of the preceding claims, ***characterized in that*** a freely rotating measuring wheel (32) with a shaft (30) parallel to the shaft (5) of the drive wheel (3) is mounted on the cover plate (17), with its own springloaded pressure wheel (33) like that pressure wheel (6) to the drive wheel (3), and **in that** the shaft (30) of the measuring wheel (32) on the underside of the cover plate (17) carries a driving wheel (15) for a receiving wheel (31) on the upper side of the drive unit (18), to which it can be coupled in a torque-locking manner when the cover plate (17) is placed on the housing (1), and which receiving wheel (31) carries the counting disc (12) with its shaft inside the drive unit (18).

8. Apparatus according to one of the preceding claims, ***characterized in that*** the apparatus and its electronic control unit (10) can be operated via an electric cable (37) by the remote control unit (16)

9. Apparatus according to one of claims 4 to 8, ***characterized in that*** the torque-locking connection from the driving wheel (24) to the transmission wheel (25) is realized by an axial interlocking thereof, just as the torque-locking connection of the driving wheel (15) on the lower side of the cover plate (17) to the receiving wheel (31) on the upper side of the drive unit (18), which with its shaft (48) carries the counting disc (12) inside the drive unit (18).

10. Apparatus according to claim 9, ***characterised in that*** the torque-locking connection from the driving wheel (24) to the transmission wheel (25) and the torque-locking connection from the driving wheel (15) to the receiving wheel (31) are realised **in that** the respective one wheel has axial cams (46) which, when the cover plate (17) is placed on the housing (1), engage in recesses (47) in the adjoining wheel (25, 31).

11. Apparatus according to one of the preceding claims, ***characterised in that*** the power supply (9) is ensured by a number of rechargeable batteries inside the drive unit (18), which can be charged via the electric cable (37).

12. Apparatus according to one of the preceding claims, ***characterised in that*** at least the following functions can be switched and implemented with the control unit (16) and the electronic control (10) connected thereto in the drive unit (18):
• switching on and off the device for connecting or disconnecting the power supply and switching on the laser for the counting disc (12)
• Rotary knob (41) for continuous or discrete selection of the conveying speed of the cable (43)
• Selection of the conveying line to be run
• Ready light (46) for starting the unit
• Display of the selected conveying speed on the control unit (16)
• Start button (38) to start the conveying
• Stop button (39) for stopping the delivery
• Display (40) of the travelled conveying distance
• Restart button (44) for resetting the displayed conveying distance
• Battery charge indicator (49) for battery power supply
• Automatic shutdown of the catheter laser when delivery is stopped via control cable to the laser generator
• Stopping the conveyor when the selected conveyor section has been travelled and emitting a signal tone.

## Revendications

1. Appareils pour le transport contrôlé d'un câble sous forme de cathéter avec un guide de lumière ou respectivement une fibre optique ou un câble électrique ou un tube en forme de câble, qui comprend une roue d'entraînement (3) et une roue de pression (6) pouvant être pressée radialement sur celle-ci, entre lesquelles le câble (43) à transporter peut être serré, de sorte qu'il peut être transporté par entraînement de la roue d'entraînement (3) par une unité d'entraînement (18) avec unité de commande (16) et commande électronique associées, et le mouvement de rotation et la vitesse de rotation de la roue d'entraînement (3) peuvent être mesurés au moyen d'un dispositif de mesure
***caractérisé en ce que***
le dispositif est construit en trois parties, avec d'une part une plaque de recouvrement (17) stérilisable à au moins 180°C, sur laquelle sont montées extérieurement la roue d'entraînement (3) et la roue de pression (6) pouvant être pressée radialement contre elle, et d'autre part une unité d'entraînement (18) non stérilisable avec commande électronique (10), moteur électrique (8), réducteur (11) et alimentation en courant (9), et troisièmement un boîtier (1) en forme de boîte, stérilisable à au moins 180°C, dans lequel l'unité d'entraînement (18) peut être complètement insérée et peut être fermée de manière stérile avec la plaque de recouvrement (17), de sorte que le dispositif est complètement stérile de tous les côtés depuis l'extérieur, et une unité de commande (16) associée qui peut être reliée au dispositif stérilisé par un câble électrique (37).

2. Appareil selon la revendication 1, ***caractérisé en ce que*** le mouvement de rotation de la roue d'entra nement (3) est transmis à un disque de comptage (12) tournant en synchronisme avec celui-ci et présentant des fentes (13) ou des trous radiaux dans la zone périphérique, qui peut être mis en rotation par une barrière lumineuse fixe, pour déterminer le mouvement et la vitesse de rotation de la roue d'entra nement (3). respectivement un guide de lumière.

3. Dispositif selon l'une des revendications précédentes, ***caractérisé en ce que*** le boîtier (1) présente sur sa face extérieure (2) une roue d'entraînement (3), avec une roue de pression (6) qui agit sur sa surface périphérique (4) dans la direction radiale, qui est déplaçable perpendiculairement à son axe (5) et qui présente un axe (7) monté parallèlement à celui de la roue d'entraînement (3), de sorte que le câble (43) puisse être serré entre la roue d'entraînement (3) et la roue de pression (6) pour le transport, et que l'unité d'entraînement (18) comporte un moteur électrique (8) avec alimentation électrique (9) et commande électronique (10) associées à l'intérieur du boîtier (1), par lequel la roue d'entraînement (3) peut être entraînée par un réducteur (11), et que dans le boîtier (1) se trouve un disque de comptage (12) avec des fentes (13) ou des trous radiaux dans sa région périphérique (14), qui peut être traversé par une barrière lumineuse fixe, pour détecter le mouvement et la vitesse de rotation du disque de comptage (12) de la roue de mesure (32) et donc de la roue d'entraînement (3), pour générer des signaux électriques à traiter et à afficher sur une unité de commande associée (16) qui peut être connectée au moyen d'un câble électrique (37) et à partir de laquelle l'ensemble du dispositif peut être commandé.

4. Appareil selon l'une des revendications précédentes, ***caractérisé en ce que*** le moteur électrique (8) à l'intérieur de l'unité d'entraînement (18) est relié par son arbre de sortie à un arbre (22) portant un engrenage conique (20) qui est en prise avec un engrenage (21) sur l'arbre (23) de la roue motrice (24) située à l'extérieur de l'unité d'entraînement (18), qui à son tour peut être mis en prise par blocage du couple avec une roue de transmission (25) située sur la face inférieure de la plaque de recouvrement (17), cette roue de transmission (25) étant placée sur l'arbre (5) de la roue motrice (3) située à l'extérieur de la plaque de recouvrement (17).

5. Appareil selon l'une des revendications précédentes, ***caractérisé en ce que*** la roue de pression mobile (6) appartenant à la roue d'entraînement (3) présente à l'extérieur sur la plaque de recouvrement (17) un arbre (7) qui traverse la plaque de recouvrement (17) dans un évidement (27), et cet arbre (7) est guidé de manière mobile à l'écart de la roue d'entraînement (3) contre la force de ressorts (29) sur un coulisseau (28) sur la face inférieure de la plaque de recouvrement (17).

6. Appareil selon l'une des revendications 4 à 5, ***caractérisé en ce que*** la roue motrice (3) porte une roue de transmission (25) à l'extérieur sur la plaque de recouvrement (17) avec son arbre (5) sur la face inférieure de la plaque de recouvrement (17), pour l'accouplement à couple avec la roue motrice (24) sur le dessus de l'unité d'entraînement (18) lorsque la plaque de recouvrement (17) est placée sur le boîtier (1).

7. Appareil selon l'une des revendications précédentes, ***caractérisé en ce qu'**une* roue de mesure (32) tournant librement avec un arbre (30) parallèle à l'arbre (5) de la roue d'entraînement (3) est montée sur la plaque de recouvrement (17), avec sa propre roue de pression (33) chargée par ressort comme cette roue de pression (6) à la roue d'entraînement (3), et **en ce que** l'arbre (30) de la roue de mesure (32) sur la face inférieure de la plaque de recouvrement (17) porte une roue d'entraînement (15) pour une roue de réception (31) sur la face supérieure de l'unité d'entraînement (18), à laquelle elle peut être accouplée de manière solidaire en couple lorsque la plaque de recouvrement (17) est placée sur le boîtier (1), et laquelle roue de réception (31) porte le disque de comptage (12) avec son arbre à l'intérieur de l'unité d'entraînement (18).

8. Appareil selon l'une des revendications précédentes, ***caractérisé en ce que*** l'appareil et son unité de commande électronique (10) peuvent être commandés par l'unité de télécommande (16) via un câble électrique (37).

9. Appareil selon l'une des revendications 4 à 8, ***caractérisé en ce que*** la liaison par couple de la roue motrice (24) à la roue de transmission (25) est réalisée par un emboîtement axial de celle-ci, tout comme la liaison par couple de la roue motrice (15) sur la face inférieure de la plaque de recouvrement (17) à la roue réceptrice (31) sur la face supérieure de l'unité d'entraînement (18), qui avec son arbre (48) porte le disque de comptage (12) à l'intérieur de l'unité d'entraînement (18).

10. Appareil selon la revendication 9, ***caractérisé en ce que*** la liaison par couple de la roue motrice (24) à la roue de transmission (25) et la liaison par couple de la roue motrice (15) à la roue réceptrice (31) sont réalisées **en ce que** chaque roue présente des cames axiales (46) qui, lorsque la plaque de recouvrement (17) est placée sur le boîtier (1), s'engagent dans des évidements (47) de la roue adjacente (25, 31).

11. Appareil selon l'une des revendications précédentes, ***caractérisé en ce que*** l'alimentation électrique (9) est assurée par un certain nombre de batteries rechargeables à l'intérieur de l'unité d'entraînement (18), qui peuvent être chargées par le câble électrique (37).

12. Appareil selon l'une des revendications précédentes, *caractérisé en ce qu*'au moins les fonctions suivantes peuvent être commutées et réalisées avec l'unité de commande (16) et la commande électronique (10) qui lui est reliée dans l'unité d'entraînement (18):
• la mise en marche et l'arrêt du dispositif de connexion ou de déconnexion de l'alimentation électrique et la mise en marche du laser pour le disque de comptage (12),
• Bouton rotatif (41) pour la sélection continue ou discrète de la vitesse de transport du câble (43),
• Sélection de la ligne de transport à utilizer,
• Lumière prête (46) pour le démarrage de l'unité,
• Affichage de la vitesse de transport sélectionnée sur l'unité de commande (16),
• Bouton de démarrage (38) pour démarrer le convoyage,
• Bouton d'arrêt (39) pour arrêter la livraison,
• Affichage (40) de la distance de transport parcourue,
• Bouton de redémarrage (44) pour réinitialiser la distance de transport affichée,
• Indicateur de charge de la batterie (49) pour l'alimentation par batterie,
• Arrêt automatique du laser du cathéter lorsque la livraison est arrêtée par le câble de commande du générateur laser,
• Arrêt du convoyeur lorsque la section de convoyeur sélectionnée a été parcourue et émission d'un signal sonore.
